# EUROPEAN PATENT SPECIFICATION

(11) **EP 2 665 714 B1**
(45) Date of publication and mention of the grant of the patent: **13.04.2016**
(21) Application number: 12705314.8
(22) Date of filing: 18.01.2012
(51) Int. Cl.: C07D 301/12, C07D 303/42

(54) **PROCESS FOR EPOXIDATION OF VEGETABLE OILS**
VERFAHREN ZUR EPOXIDIERUNG VON PFLANZENÖLEN
PROCÉDÉ POUR L'ÉPOXYDATION D'HUILES VÉGÉTALES

(30) Priority: 18.01.2011 FI 20115047
(43) Date of publication of application: 27.11.2013
(73) Proprietor: Raisio plc, 21200 Raisio (FI)
(72) Inventor: LÄMSÄ, Merja, FI-21160 Merimasku (FI); KOSONEN, Kaisa, FI-20610 Turku (FI)
(74) Representative: Lalli, Esko
(86) International application number: PCT/FI2012/050042
(87) International publication number: WO 2012/098295

(56) References cited:
- US-A- 6 051 725
- STANISLAV&EMSP14;A. GRABOVSKIY ET AL: "Epoxidation of Polyunsaturated Fatty Acid Double Bonds by Dioxirane Reagent: Regioselectivity and Lipid Supramolecular Organization", HELVETICA CHIMICA ACTA, vol. 89, no. 10, 1 October 2006 (2006-10-01), pages 2243-2253, XP55023350, ISSN: 0018-019X, DOI: 10.1002/hlca.200690209

## Description

### Field of the invention

The present invention relates to a novel process for epoxidation of vegetable oils.

### Background

Traditionally, many products of the chemical industry have been based on mineral oils. As the demand for products based on renewable raw materials raises, vegetable oils have become an important raw material in the chemical industry. However, for several applications many chemical and biochemical reactions are needed in order to converse raw vegetable oils to functional products. Epoxidation is among those reactions that have been widely used for modification of unsaturated oils.

Industrial production of epoxidized organic compounds usually requires the presence of hydrogen peroxide, percarboxylic acids and strong acids and is carried out in an organic solvent. The most extensively epoxidized oil is soybean oil which is generally produced with peroxyformic or peroxyacetic acid generated *in situ* from hydrogen peroxide and the appropriate alkanoic acid and sometimes in the presence of a solvent. According to GB927048, soya bean oil is epoxidized by reacting the oil with an epoxidizing agent which is a mixture of saccharide or polyalcohol and hydrogen peroxide, in the presence of an acidic activator. In DE19903700, epoxidation of rapeseed oil requires formic acid, hydrogen peroxide and phosphoric acid. Hydrogen peroxide is also needed in a two-phase catalysis with organometallic compounds for epoxidation of vegetable oils (Jiang et al., J. Am. Oil Chem. Soc, 2010, 87, 83-91).

In high concentrations, hydrogen peroxide is an aggressive oxidizer and will corrode many materials. Industrial use of this hazardous material thus involves risks and is not environmentally acceptable. Percarboxylic acids, which also are strong oxidizing agents, as well as the strong acids involved in the current epoxidation processes have the same problematic properties as hydrogen peroxide, thus causing safety concerns and corrosion problems.

US 6051725 discloses the epoxidation of castor oil and an unsaturated derivate thereof called Castung^{®} oil. The epoxidation is mainly performed using hydrogen peroxide but also monopersulfate compounds, such as Oxone^{®}, which is potassium monoper-oxysulfate, can be used as oxidation agents. The patent teaches the use of phase-transfer catalysts such as methyltri-n-octylammonium diperoxotungstophosphate and crown ethers to accomplish the epoxidation reaction. The purification of the end product is therefore complicated. The degree of epoxidation also remains quite low. Preferably organic solvents, such as toluene, hexane and chlorinated hydrocarbons are used.

Consequently, there exists a need for an improved environmentally acceptable epoxidation process of vegetable oils wherein the use of strong acids and hydrogen peroxide is avoided. The process should also be economically feasible and provide good yields and further be capable of producing a high enough conversion of the carbon-carbon double bonds to epoxide groups.

### Summary of the invention

It has now been found that epoxidation of vegetable oils is efficiently carried out with peroxymonosulfate in a pH-controlled aqueous solution in the presence of a ketone, but in the absence of a phase-transfer catalyst. This reaction is selective without adverse side reactions and the epoxidation degree (the percentage of double bonds epoxidized) can be controlled. Moreover, the process can be realised very conveniently because the use of different chemicals and organic solvents can be reduced, or even totally avoided compared to prior known methods.

It is therefore an object of the present invention to provide a process for epoxidation of vegetable oil wherein vegetable oil is reacted with peroxymonosulfate in a pH-controlled aqueous ketone solution without the use of a phase-transfer catalyst.

Another object of the invention is a process for preparing a totally or practically totally epoxidized, light-coloured and pure product, using as raw material vegetable oil, especially crude rapeseed oil.

Other objects, features and advantages of the present invention will become apparent from the following detailed description. It should be understood, however, that the detailed description and the specific examples, while indicating preferred embodiments of the invention, are given for illustration only.

### Detailed description of the invention

The present invention relates to a novel process for epoxidation of vegetable oils. The epoxidation process according to the present invention is characterized in that vegetable oil is reacted with peroxymonosulfate in a pH-controlled aqueous solution in the presence of a ketone but in the absence of a phase transfer catalyst.

Any vegetable oil which has a content of unsaturated fatty acids of at least 70 %, preferably at least 80 %, and most preferably at least 90 % may be used as starting material in the process according to the invention. The iodine number of the vegetable oil may vary from 75 to 200, preferably from 80 to 200. The vegetable oils, which can be used as a substrate in the epoxidation include for example rapeseed oil, safflower oil, camelina oil (oil from Camelina sativa, also known as false flax, linseed dodder or gold-of-pleasure), linseed oil (also known as flax seed oil), high-oleic sunflower oil, soybean oil, sunflower oil, corn oil and olive oil. The most preferred vegetable oil for use in the process according to the invention is rapeseed oil, preferably crude rapeseed oil. Also crude versions of the other suitable vegetable oils are applicable as starting material. Other preferred vegetable oils for use in the process of the present invention are camelina oil and high-oleic sunflower oil. Also mixtures of oils can be utilised.

Rapeseed oil is rich in unsaturated fatty acids, such as oleic acid, linoleic acid and alpha-linolenic acid. It is recommended as a healthy nutritive oil due to its beneficial fatty acid profile. Rapeseed oil has also many non-food uses such as in industrial lubricants and biofuels. Crude (unrefined) rapeseed oil differs from alkali-refined, bleached and deodorized rapeseed oil in that it contains small amounts of protein or other solids, phosphatides, undesirable natural flavours and odours, free fatty acids, pigments, waxes and sulphur-containing compounds.

Camelina oil can be processed using normal refining treatments. Camelina oil is very rich in unsaturated fatty acids, the content of unsaturated fatty acids in the oil is approximately 90 %. Linoleic acid and alpha-linolenic acid constitute over 50 % of the total fatty acids. Unlike most vegetable oils, camelina oil is higher in linolenic acid than linoleic acid. Camelina oil is an excellent source of essential omega-3 fatty acids. Camelina oil is used in cosmetics and soaps, and has a potential for biofuel.

High oleic sunflower oil is very high in monounsaturated fatty acid having a minimum of 75 %, preferably of 80 % oleic acid, and most preferably at least 90 % oleic acid. Research has been leading to the development of varieties that may exceed 95 % oleic fatty acid content. High oleic sunflower oil contains linoleic acid about 2-9 % and very small amounts of linolenic acid.

In the epoxidation of vegetable oil, the double bonds of vegetable oil form epoxide rings with oxygen atoms. In the present invention, an epoxidizing intermediate (dioxirane) is formed *in situ* from the reaction of ketone with peroxymonosulfate at a suitable pH value e.g. under buffered conditions. Said intermediate is used for oxygen transfer to the carbon-carbon double bonds, an epoxidized product is formed and the initial ketone is thereby regenerated. Depending on the amount of double bonds epoxidized, epoxidized vegetable oils with various epoxidization degrees (i.e. the ratio of epoxidized carbon-carbon double bonds after the reaction to the total carbon-carbon double bonds available before the reaction) are obtained. The epoxidation degree can finally be controlled by adjusting the reaction conditions such as temperature, amount of reagents and reaction time.

The desired epoxidation degree depends on the application of use of the epoxidized oil. Epoxidized vegetable oils are used e.g. as plasticizers and stabilizers of PVC and are valuable materials in polymer science with application possibilities as rubbers, resins and coatings. The degree of epoxidation will influence the properties of the polymer, such as viscosity and melting temperature. The process according to the present invention preferably provides epoxidation degrees from 50 to 100 %, more preferably from 70 to 100 %, still more preferably from 80 to 100 %, further more preferably from 90 to 100 %, and most preferably from 95 to 100 %.

In the process according to the present invention, peroxymonosulfate is used as the oxidizing agent. A preferred peroxymonosulfate is potassium peroxymonosulfate (KHSO₅). As potassium peroxymonosulfate is rather unstable as such, it is preferably present as a component of a triple salt, the triple salt preferably consisting of potassium peroxymonosulfate, potassium hydrogen sulfate and potassium sulfate. Such a triple salt is commercially available e.g. under the trade names Oxone ® and Caroat®. Also other peroxymonosulfates, such as sodium peroxymonosulfate and ammonium peroxymonosulfate which can be formed *in situ,* are applicable. Also potassium peroxymonosulfate may be produced *in situ.*

The epoxidation of vegetable oils according to the present invention requires the presence of a ketone. In the presence of a ketone, the peroxymonosulfate generates *in situ* dioxiranes which are known to be powerful epoxidation species. The ketone is preferably a C₃-C₄ ketone. Among C₃-C₄ ketones i.e. CH₃COCH₃, CH₃CH₂COCH₃ and CH₃COCOCH₃, acetone is most preferred for the purposes of the present invention. The amount of acetone in the reaction mixture has a direct impact on the epoxidation degree.

The reaction is carried out in a pH-controlled aqueous solution preferably without an organic solvent. The proportions of the reagents may vary but generally the molar ratio of peroxymonosulfate to carbon-carbon double bond in the substrate can vary from 0.50:1 to 2.0:1, and more preferably it is at least 0.60:1. The molar ratio of ketone to peroxymonosulphate is preferably chosen to be from 1.0:1 to 12:1, more preferably from 2.0:1 to 12:1, and most preferably from 2.0:1 to 5.0:1.

Without pH adjustment the pH of the reaction mixture is too acidic for the reaction to take place. At higher pH values (pH>9), peroxymonosulfate decomposes in solution, at lower pH values its reactivity may decrease. It is therefore important to control and adjust the pH value of the reaction mixture and thus inhibit ring-opening side reactions that adversely affect the stability of the oxirane rings. The pH of the reaction mixture is therefore adjusted to pH 6-9, preferably to pH 7-8. Suitable raw materials for buffers for adjusting the pH are for example alkali metal carbonates, alkaline earth metal carbonates, alkali metal hydrogen carbonates, alkali metal hydroxides, alkali metal hydrogen phosphates, alkali metal dihydrogen phosphates and mixtures thereof. Preferred buffers include alkali metal hydrogen phosphates, alkali metal dihydrogen phosphates and mixtures thereof. The use of buffer is however not necessary. The pH value can be kept at the optimal level by adding a neutralising agent such as an alkali metal hydroxide during the reaction or an alkali metal hydrogen carbonate preferably already before or when starting the reaction. Also a combination of these two types of neutralising agents can be used.

The reaction does not require high pressure but is realizable under normal pressure (101 kPa). The reaction is performed at a temperature from 10 to 40 °C, preferably from 15 to below 30 °C or to 30 °C, and most preferably from 15 to 25 °C. It has now been realised the obtained epoxidation degree is highly dependent on the reaction temperature.

The presence of a phase-transfer catalyst is not needed in the process according to the current invention, *i.e.* the process is phase-transfer catalyst-free. In addition to increasing costs, the use of phase-transfer catalysts leads to additional purification steps at the end of the process in order to remove the catalyst. Examples of typical phase-transfer catalysts are crown ethers, quaternary ammonium or phosphonium salts, such as methyl trioctyl ammonium chloride or methyl tri-n-octyl ammonium(diperoxotungsto) phosphate.

Furthermore, as the process according to the invention preferably is solvent-free, also the costs and purification steps caused by the use of an organic solvent are avoided. The process therefore can be performed in the absence of an organic solvent, i.e. it is solvent-free or substantially solvent-free. "Substantially solvent-free" or "substantially without an organic solvent" means that an organic solvent is not actively introduced into the reaction mixture for solvent purposes but may be present in minor amounts as an impurity e.g. at most 2 % by weight solvent of the reaction mixture. However, the C₃-C₄ ketone which function as a precursor of the epoxidizing reagent and not as a solvent in the process according to the invention are within this disclosure excluded from the definition of organic solvent. Typical examples of organic solvents as meant in the present invention are toluene, hexane, chlorinated hydrocarbons such as dichloromethane and trichloromethane, alcohols such as methanol and ethanol, and acetonitrile. Thus, this kind of organic solvents are preferably not used in the present invention.

The reaction time in the process according to the invention depends on the desired epoxidation degree of the end product and may vary for example between 2 to 8 hours, depending on the production scale and the equipment used. A preferred reaction time is approximately 6 hours.

The conversion to epoxides can be monitored by Fourier transform infrared spectrometry, NMR spectrometry and a titration method (oxirane oxygen content).

Stirring of the reaction mixture is very important for the proper outcome of the reaction. The aim of stirring in the present invention is to maximize the reactive area of the components by dispersing the fat droplets of the vegetable oil as effectively as possible among the aqueous ketone solution. In laboratory scale this is achieved by vigorous mechanical stirring. For the reaction to take place e.g. conventional efficient mixing of the reaction mixture is relevant.

It has also been found that it is advantageous to add the peroxymonosulfate continuously or in several portions (typically at least 10, more typically at least 50 and most typically at least 100 addition times) during the process. In practice, vegetable oil (the substrate to be epoxidized) and ketone are mixed together and the pH is adjusted with a sufficient amount of buffer and/or alkali to inhibit ring-opening side reactions during the epoxidation. The peroxymonosulfate is then added slowly into a stirred mixture at room temperature. The reaction may still be continued after ending the addition of oxidising agent. The phases are thereafter allowed to settle and then the upper layer is separated. The upper layer containing mainly (e.g. more than 90 % by weight) the epoxidized product is then washed, preferably with water, and finally dried. If necessary, before drying any solid residues are removed for example by filtration, centrifugation or decantation. Drying can be performed by any known techniques such as by distillation or evaporation, e.g. by introducing vacuum. There is preferably no need for any solvent extractions of the oil phase to refine the epoxidized product.

With the process according to the invention it is possible to produce practically totally (epoxidation degree of 97-99.9 %) or even totally epoxidized, pure and light-coloured products using vegetable oils, especially crude rapeseed oil, as raw material. Even if using e.g. crude rapeseed oil the peroxymonosulfate, acetone and water are able to purify the epoxidized vegetable oil during the process to a pure and nice looking product.

Most preferably the process according to the invention is performed in a reaction mixture consisting of the following components: vegetable oil, water, pH-regulating agent(s), a ketone, and a composition containing or producing peroxymonosulfate. The recovery needs no additional chemicals, preferably only water is used. Using so few components in the epoxidation reaction and in the recovery of the epoxidized vegetable oil makes the process as a whole very simple and easy to accomplish on an industrial scale.

The selectivity and good yields of the process according to the invention are due to the specific reagents chosen and preferably also to the reaction conditions of the process. Good yields are obtained because unnecessary process and/or purification steps can be avoided. The absence of a phase-transfer catalyst and preferably also the absence of an organic solvent as defined herein makes the separation of the product easy. Unwanted side reactions are avoided. The epoxidation process according to the invention is extremely neat and produces practically no by-products. The reagents are low-cost, commercially available and environmentally benign. The hazardous handling of peracids, hydrogen peroxide and strong acids is successfully avoided in the present epoxidation process. Also the troublesome use of phase-transfer catalysts is successfully avoided. The process according to the present invention is therefore cost effective, technically simple, industrially easy to perform, is able to produce epoxidized vegetable oils with high conversions of carbon-carbon double bonds to epoxy-groups, and excellent yields of epoxidized vegetable oil can be obtained.

### Example 1

20 g of crude rapeseed oil and 10.4 g of NaHCO₃ were slurried into 14.4 g of methylethylketone with vigorous stirring in a 11 three-necked flask. The flask was equipped with a mechanical stirrer, condenser and addition funnel containing an aqueous solution of peroxymonosulfate (30.4 g of 2KHSO₅·KHSO₄·K₂SO₄ in 350 ml of water). Vigorous stirring was continued at room temperature and peroxymonosulfate solution was added dropwise over the course of 3.5 hours. The upper organic layer was separated, washed with water and dried over anhydrous Na₂SO₄. Finally methylethylketone was distilled off at 65 °C and 70 mmHg for 20 minutes. The oxirane oxygen content (AOCS Cd9-57) was 3.6 % (54 % of theoretical). ¹H NMR spectrum of the product revealed that 53 % conversion of the carbon-carbon double bonds to epoxide groups had been taken place without any side reactions. The obtained epoxidized rapeseed oil was pure and light-coloured.

### Example 2

20 g of refined camelina oil and 69.1 g of acetone were charged into a 11 three-necked flask containing phosphate buffer solution (16.7 g of KH₂PO₄ and 83.1 g of K₂HPO₄ in 600 ml of water). The flask was equipped with a mechanical stirrer, condenser and addition funnel containing an aqueous solution of peroxymonosulfate (73.2 g of 2KHSO₅·KHSO₄·K₂SO₄ in 320 ml of water). The mixture was stirred vigorously at room temperature. Vigorous stirring was continued and peroxymonosulfate solution was added dropwise over the course of 2 hours. The stirring was continued for additional 3 hours. The upper layer was separated, washed with water and dried over anhydrous Na₂SO₄. The oxirane oxygen content (AOCS Cd9-57) was 7.7 % (89 % of theoretical). ¹H NMR spectrum of the product revealed that 90 % conversion of the carbon-carbon double bonds to epoxide groups had been taken place without any side reactions. The obtained epoxidized camelina oil was pure and light-coloured.

### Example 3

In this example the procedure of Example 2 was followed. Therefore, the molar ratios peroxymonosulfate to carbon-carbon double bond and acetone to peroxymonosulfate were the same as in Example 2. As substrate high-oleic sunflower oil was used instead of camelina oil and the reaction mixture was stirred at 35°C instead of at 22 °C. 20 g of refined high-oleic sunflower oil and 38.4 g of acetone were charged into a 11 three-necked flask containing phosphate buffer solution (16.7 g of KH₂PO₄ and 83.1 g of K₂HPO₄ in 600 ml of water). The flask was equipped with a mechanical stirrer, condenser and addition funnel containing an aqueous solution of peroxymonosulfate (40,6 g of 2KHSO₅·KHSO₄·K₂SO₄ in 180 ml of water). The mixture was stirred vigorously at 35°C. Vigorous stirring was continued and peroxymonosulfate solution was added dropwise over the course of 2 hours. The stirring was continued for additional 3 hours. The upper layer was separated, washed with warm (50°C) water and dried over anhydrous Na₂SO₄. The conversion was 74 % of theoretical (oxirane oxygen content was 3.7 %). ¹H NMR spectrum of the product confirmed that 73 % conversion of the carbon-carbon double bonds to epoxide groups had been taken place without any side reactions. The obtained epoxidized high-oleic sunflower oil was pure and light-coloured.

### Example 4

Two similar reactions were performed, but at different temperatures (at 5 °C and at 22 °C). In both reactions 20 g of crude rapeseed oil and 38.9 g of acetone were charged into a 11 three-necked flask containing a phosphate buffer solution (78 g of NaH₂PO₄·2H₂O and 8.6 g of NaOH in 450 ml of water). The flask was equipped with a mechanical stirrer, condenser and addition funnel. The content of the flask was kept at 5°C with an ice bath. Refrigerated (4 °C) aqueous solution of peroxymonosulfate (41.1 g of 2KHSO₅·KHSO₄·K₂SO₄ in 90 ml of water) was added dropwise to a vigorously stirred reaction mixture over the course of 1.5 hours and the stirring was continued for additional 4 hours at 5°C. The other reaction was performed at room temperature with all reagents at room temperature. The layers were separated, the upper layer was washed with water and dried over anhydrous Na₂SO₄. The oxirane oxygen contents (AOCS Cd9-57) were measured. The results are presented in Table 1.

**Table 1. Results of a comparison between the process at two different temperatures**

| temperature (°C) | oxirane oxygen content % | % of theoretical |
|---|---|---|
| 5 | 1.9 | 29 |
| 22 | 5.7 | 86 |

As can be seen from the results a reaction temperature of close to 0 °C does not lead to an acceptable epoxidation degree. However, at room temperature the reaction proceeds well.

### Example 5

To a 11 three-necked flask were added 40 g of crude rapeseed oil, 200 ml of water and 103.6 g of acetone and the mixture was stirred vigorously at room temperature. An aqueous solution of peroxymonosulfate (54.8 g of 2KHSO₅·KHSO₄·K₂SO₄ in 240 ml of water) was added dropwise over the course of 4 hours and stirring was continued for additional 2 hours. To adjust pH to 7 a 2 M KOH solution was added dropwise into the stirred mixture and the addition was continued for all the reaction time. The upper layer was separated, washed and dried over anhydrous Na₂SO₄. The oxirane oxygen content (AOCS Cd9-57) was 5.3 % (79 % of theoretical). The obtained epoxidized rapeseed oil was pure and light-coloured.

### Example 6

In this example the procedure of Example 2 was followed. Therefore, the molar ratios peroxymonosulfate to carbon-carbon double bond and acetone to peroxymonosulfate were the same as in Example 2. As substrate rapeseed oil was used instead of camelina oil. 20 g of crude rapeseed oil and 51.7 g of acetone were charged into a 11 three-necked flask containing phosphate buffer solution (16.7 g of KH₂PO₄ and 83.1 g of K₂HPO₄ in 600 ml of water). The flask was equipped with a mechanical stirrer, condenser and addition funnel containing an aqueous solution of peroxymonosulfate (54.7 g of 2KHSO₅·KHSO₄·K₂SO₄ in 250 ml of water). The mixture was stirred vigorously at room temperature. The vigorous stirring was continued and peroxymonosulfate solution was added dropwise over the course of 2 hours. The stirring was continued for additional 4 hours at room temperature. The upper layer was separated, washed with water and dried over anhydrous Na₂SO₄. The oxirane oxygen content (AOCS Cd9-57) was 6.6 % (99 % of theoretical). The obtained epoxidized rapeseed oil was pure and light-coloured.

This example in comparison to Examples 2 and 3 shows that the present process is especially suitable for producing epoxidized rapeseed oil.

## Claims

1. A process for epoxidation of vegetable oil wherein vegetable oil is reacted with peroxymonosulfate in a reaction mixture comprising a pH-controlled aqueous ketone solution, wherein the reaction mixture has a pH-value of 6 to 9 and has a temperature of 10 to 40 °C, **characterised in that** the reaction mixture is free of a phase-transfer catalyst.

2. The process according to claim 1, wherein the peroxymonosulfate is selected from the group consisting of potassium peroxymonosulfate, sodium peroxymonosulfate and ammonium peroxymonosulfate.

3. The process according to claim 1 or 2, wherein the ketone is a C₃-C₄ ketone, preferably acetone.

4. The process according to any one of claims 1 to 3, wherein the vegetable oil is selected from the group consisting of rapeseed oil, safflower oil, camelina oil, linseed oil, high oleic sunflower oil, soybean oil, sunflower oil, corn oil and olive oil, preferably rapeseed oil.

5. The process according to any one of claims 1 to 4, wherein the reaction mixture has a pH-value from 7 to 8.

6. The process according to any one of claims 1 to 5, wherein the epoxidation reaction is driven so far that 50 to 100 % of the double bonds of the vegetable oil are epoxidized.

7. The process according to any one of claims 1 to 6, wherein the molar ratio of peroxymonosulfate to carbon-carbon double bond in the vegetable oil is from 0.50:1 to 2.0:1.

8. The process according to any one of claims 1 to 7, wherein the molar ratio of ketone to peroxymonosulfate is from 1.0:1 to 12:1.

9. The process according to any one of claims 1 to 8, wherein the temperature of the reaction mixture is from 15 to 25 °C.

10. The process according to any one of claims 1 to 9, wherein the reaction time is from 2 to 8 hours.

11. The process according to any one of claims 1 to 10, wherein the reaction mixture comprises substantially no organic solvent.

12. The process according to any one of claims 1 to 11, wherein the epoxidized vegetable oil is recovered from the reaction mixture by using at least the following steps:
- separating the oil phase comprising epoxidized vegetable oil,
- washing the oil phase with water,
- optionally removing any solid residues from the oil phase,
- removing any ketone and/or water residues from the oil phase, to obtain the epoxidized vegetable oil.

13. The process according to claim 12, wherein the oil phase is substantially solvent-free.

14. The process according to claim 12 or 13, wherein the recovery step does not include solvent extraction of the epoxidized vegetable oil from the oil phase.

## Patentansprüche

1. Verfahren zum Epoxidieren von Pflanzenöl, wobei Pflanzenöl in einem Ansatz, der eine pH-kontrollierte wässrige Ketonlösung umfasst, mit Peroxymonosulfat umgesetzt wird, wobei der Ansatz einen pH-Wert von 6 bis 9 und eine Temperatur von 10 bis 40°C aufweist, **dadurch gekennzeichnet, dass** der Ansatz frei von einem Phasentransferkatalysator ist.

2. Verfahren nach Anspruch 1, wobei das Peroxymonosulfat aus der Gruppe bestehend aus Kaliumperoxymonosulfat, Natriumperoxymonosulfat und Ammoniumperoxymonosulfat ausgewählt ist.

3. Verfahren nach Anspruch 1 oder 2, wobei das Keton ein C₃-C₄-Keton, vorzugsweise Aceton, ist.

4. Verfahren nach einem der Ansprüche 1 bis 3, wobei das Pflanzenöl aus der Gruppe bestehend aus Rapsöl, Safloröl, Camelinaöl, Leinöl, Sonnenblumenöl mit hohem Ölsäuregehalt, Sojaöl, Sonnenblumenöl, Maiskeimöl und Olivenöl ausgewählt ist und vorzugsweise Rapsöl ist.

5. Verfahren nach einem der Ansprüche 1 bis 4, wobei der Ansatz einen pH-Wert von 7 bis 8 aufweist.

6. Verfahren nach einem der Ansprüche 1 bis 5, wobei die Epoxidation so weit vorangetrieben wird, dass 50 bis 100% der Doppelbindungen des Pflanzenöls epoxidiert werden.

7. Verfahren nach einem der Ansprüche 1 bis 6, wobei das Molverhältnis von Peroxymonosulfat zu Kohlenstoff-Kohlenstoff-Doppelbindung in dem Pflanzenöl 0,50:1 bis 2,0:1 beträgt.

8. Verfahren nach einem der Ansprüche 1 bis 7, wobei das Molverhältnis von Keton zu Peroxymonosulfat 1,0:1 bis 12:1 beträgt.

9. Verfahren nach einem der Ansprüche 1 bis 8, wobei die Temperatur des Ansatzes 15 bis 25°C beträgt.

10. Verfahren nach einem der Ansprüche 1 bis 9, wobei die Reaktionsdauer 2 bis 8 Stunden beträgt.

11. Verfahren nach einem der Ansprüche 1 bis 10, wobei der Ansatz im Wesentlichen kein organisches Lösungsmittel umfasst.

12. Verfahren nach einem der Ansprüche 1 bis 11, wobei das epoxidierte Pflanzenöl aus dem Ansatz unter Einsatz von mindestens der folgenden Schritte gewonnen wird:
- Abtrennen der epoxidiertes Pflanzenöl umfassenden Ölphase,
- Waschen der Ölphase mit Wasser,
- gegebenenfalls Entfernen von Feststoffresten aus der Ölphase,
- Entfernen von Keton- und/oder Wasserresten aus der Ölphase, wodurch man das epoxidierte Pflanzenöl erhält.

13. Verfahren nach Anspruch 12, wobei die Ölphase im Wesentlichen lösungsmittelfrei ist.

14. Verfahren nach Anspruch 12 oder 13, wobei der Gewinnungsschritt keine Lösungsmittelextraktion des epoxidierten Pflanzenöls aus der Ölphase beinhaltet.

## Revendications

1. Procédé d'époxydation d'huile végétale, dans lequel l'huile végétale est réagie avec du peroxymonosulfate dans un mélange réactionnel comprenant une solution de cétone aqueuse à pH contrôlé, où le mélange réactionnel possède une valeur de pH allant de 6 à 9 et possède une température allant de 10 à 40°C, **caractérisé en ce que** le mélange réactionnel est dépourvu de catalyseur à transfert de phase.

2. Procédé selon la revendication 1, dans lequel le peroxymonosulfate est choisi dans le groupe constitué par le peroxymonosulfate de potassium, le peroxymonosulfate de sodium et le peroxymonosulfate d'ammonium.

3. Procédé selon la revendication 1 ou 2, dans lequel la cétone est une C₃-C₄-cétone, préférablement l'acétone.

4. Procédé selon l'une quelconque des revendications 1 à 3, dans lequel l'huile végétale est choisie dans le groupe constitué par l'huile de colza, l'huile de carthame, l'huile de lin bâtard, l'huile de lin, l'huile de tournesol à haute teneur en acide oléique, l'huile de soja, l'huile de tournesol, l'huile de maïs et l'huile d'olive, préférablement l'huile de colza.

5. Procédé selon l'une quelconque des revendications 1 à 4, dans lequel le mélange réactionnel possède une valeur de pH allant de 7 à 8.

6. Procédé selon l'une quelconque des revendications 1 à 5, dans lequel la réaction d'époxydation est menée jusqu'au point où de 50 à 100% des doubles liaisons de l'huile végétale soient époxydées.

7. Procédé selon l'une quelconque des revendications 1 à 6, dans lequel le rapport molaire du peroxymonosulfate aux doubles liaisons carbone-carbone dans l'huile végétale va de 0,50:1 à 2,0:1.

8. Procédé selon l'une quelconque des revendications 1 à 7, dans lequel le rapport molaire de la cétone au peroxymonosulfate va de 1,0:1 à 12:1.

9. Procédé selon l'une quelconque des revendications 1 à 8, dans lequel la température du mélange réactionnel va de 15 à 25°C.

10. Procédé selon l'une quelconque des revendications 1 à 9, dans lequel le temps de réaction va de 2 à 8 heures.

11. Procédé selon l'une quelconque des revendications 1 à 10, dans lequel le mélange réactionnel ne comprend sensiblement aucun solvant organique.

12. Procédé selon l'une quelconque des revendications 1 à 11, dans lequel l'huile végétale époxydée est récupérée à partir du mélange réactionnel en utilisant au moins l'une des étapes suivantes :
- la séparation de la phase huileuse comprenant l'huile végétale époxydée;
- le lavage de la phase huileuse par de l'eau;
- éventuellement l'élimination de tout résidu solide à partir de la phase huileuse;
- l'élimination de tout résidu de cétone et/ou d'eau à partir de la phase huileuse, afin d'obtenir l'huile végétale époxydée.

13. Procédé selon la revendication 12, dans lequel la phase huileuse est sensiblement dépourvue de solvant.

14. Procédé selon la revendication 12 ou 13, dans lequel l'étape de récupération n'inclut pas d'extraction par un solvant de l'huile végétale époxydée à partir de la phase huileuse.
